(19) Europäisches Patentamt
European Patent Office
Office européen des brevets

(11) **EP 1 784 166 B1**

(12) **EUROPEAN PATENT SPECIFICATION**

(45) Date of publication and mention
of the grant of the patent:
**04.03.2009 Bulletin 2009/10**

(51) Int Cl.:
*A61K 9/16* (2006.01)  *A61K 9/20* (2006.01)
*A61K 9/50* (2006.01)  *A61K 31/60* (2006.01)

(21) Application number: **05792891.3**

(22) Date of filing: **31.08.2005**

(86) International application number:
**PCT/US2005/030907**

(87) International publication number:
**WO 2006/028831 (16.03.2006 Gazette 2006/11)**

(54) **COMPOSITIONS COMPRISING 5-AMINO-2-HYDROXYBENZOIC ACID AND A REDUCING SUGAR**

ZUSAMMENSETZUNGEN MIT 5-AMINO-2-HYDROXYBENZOESÄURE UND EINEM REDUZIERENDEM ZUCKER

COMPOSITIONS CONTENANT DE L'ACIDE 5-AMINO-2-HYDROXYBENZOIQUE ET UN SUCRE DE REDUCTION

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.09.2004 US 606386 P**

(43) Date of publication of application:
**16.05.2007 Bulletin 2007/20**

(73) Proprietor: **The Procter and Gamble Company**
**Cincinnati, Ohio 45202 (US)**

(72) Inventors:
• **KACZANOWSKI, Matt, John**
**Norwich, NY 13815 (US)**
• **WILLIAMS, Thomas, Daniel**
**Mason, OH 45040 (US)**
• **TROMBLEY, Kurt, Franklin**
**Loveland, OH 45140 (US)**

• **REDMAN-FUREY, Nancy, Lee**
**Smyrna, NY 13464 (US)**

(74) Representative: **Clemo, Nicholas Graham**
**Procter & Gamble Technical Centres Limited**
**Patent Department**
**Rusham Park**
**Whitehall Lane**
**Egham**
**Surrey TW20 9NW (GB)**

(56) References cited:
**EP-A- 0 474 874          WO-A-03/032952**
**US-A- 5 514 676**

• **DATABASE WPI Section Ch, Week 199813 Derwent Publications Ltd., London, GB; Class A96, AN 1998-138660 XP002369614 & JP 10 015032 A (NISSHIN FLOUR MILLING CO) 20 January 1998 (1998-01-20) cited in the application**

## Description

FIELD OF THE INVENTION

The present invention relates to providing compositions with reduced associated degradation products that may form during storage.

BACKGROUND OF THE INVENTION

The Maillard or browning reaction is initiated by a non-enzymatic condensation of a reducing sugar, such as glucose or galactose (constituents of lactose, a disaccharide), with a primary amine group, such as on a protein, to form a Schiff base; which then undergoes an Amadori rearrangement to regenerate carbonyl activity. Subsequent reactions, including but not limited to, dehydration, rearrangement, fragmentation, and further condensation reactions may yield a variety of advanced Maillard reaction products (Smith et al. (1993) Proc. Natl. Acad. Sci. USA 91, 5710-5714).

Many pharmaceutical compositions contain both a primary amine and a reducing sugar. The powder form of many pharmaceutical products may limit the degradation of components as the presence of water has been suggested as essential for the Maillard reaction to occur (Nelson and Labuza (1994) J. Food Eng. 22, 271-289). Asacol™ (also known as 5-amino-2-hydroxybenzoic acid, 5-aminosalicylic acid, or mesalamine) is used to treat ulcerative colitis, proctitis, and proctosigmoiditis. Asacol™ is also used to prevent the symptoms of ulcerative colitis from recurring. Degradation products of 5-amino-2-hydroxybenzoic acid have been reported by Jensen et al (Int. J. Pharmaceutics 88, 177-187 (1992)). Jensen et al described four polymeric species formed by oxidative self-coupling of 5-amino-2-hydroxybenzoic acid. Japanese Patent Application No. Hei 8[1998]-15032 reported that the browning of the 5-aminosalicylic acid at the time of high-temperature storage was due to vaporization of the moisture content of the additives used in the tablet, and that the browning was accelerated by the water vapor. It also reported that the browning of 5-aminosalicylic acid was preventable by continuously removing the moisture by having the dosage form contained in an air permeable wrapping material, the outside of which is packaged and sealed with a gas-impermeable wrapping material together with at least a water absorbent or moisture absorbent. However, the inventors have now discovered that Asacol™ tablets comprising 5-amino-2-hydroxybenzoic acid and lactose sugar generate degradants that are different from the polymeric species described by Jensen et al. Various governmental regulatory agencies stipulate that amounts of impurities that may accumulate in a pharmaceutical product be below certain threshold values. Therefore, there is a need to design compositions and protocols that limit the formation of impurities in compositions comprising 5-amino-2-hydroxybenzoic acid.

SUMMARY OF THE INVENTION

The inventors have discovered that pharmaceutical compositions comprising 5-amino-2-hydroxybenzoic acid and a reducing sugar, e.g., lactose, undergo degradation and produce, in the case of lactose, 5-[2-formyl-5-(hydroxymethyl)-1H-pyrrol-1-yl]-2-hydroxybenzoic acid. The inventors have developed means to contain or reduce the degradation of 5-amino-2-hydroxybenzoic acid.

In one embodiment, the invention provides for a kit comprising:

(a) at least one unit dosage form comprising a safe and effective amount of 5-amino-2-hydroxybenzoic acid and a reducing sugar; and
(b) a predetermined amount of a desiccant.

In another embodiment of the kit, the invention provides that the concentration of a degradant in the unit dosage form, which may accumulate during storage, is no more than about 0.15%, when measured according to a stability testing method recognized by International Conference on Harmonization of technical requirements for registration of pharmaceuticals.

In another embodiment of the kit, the dosage form is made via wet granulation using water.

In another embodiment of the kit, the degradant that does not accumulate above 0.15% (wt/wt) during storage is 5-[2-Formyl-5-(hydroxymethyl)-1H-pyrrol-1-yl]-2-hydroxybenzoic acid.

In another embodiment of the kit, at least one unit dosage form and the desiccant are placed in a re-sealable or re-closable container.

In another embodiment of the kit, at least one unit dosage form is placed in one or more cavities while the desiccant is placed in one or more separate but connected cavities sharing the same environment with the dosage form.

In another embodiment of the kit, the reducing sugar is selected from the group consisting of lactose, glucose, galactose, and sucrose.

In another embodiment of the kit, the reducing sugar is lactose.

In another embodiment of the kit, the desiccant is an activated desiccant.

In another embodiment of the kit, the activated desiccant is selected from the group consisting of silica gel, indicating silica gel, molecular sieve, clay, montmorillonite, activated carbon, alumina, and mixtures thereof.

In another embodiment of the kit, the desiccant is silica gel or indicating silica gel.

In another embodiment, the invention provides for a method of making a pharmaceutical composition comprising a safe and effective amount of 5-amino-2-hydroxybenzoic acid and a reducing sugar, that does not accumulate a degradant in the amount more than about 0.15%, during its about 25°C / 60% relative humidity shelf-life; comprising: wet granulating 5-amino-2-hydroxybenzoic acid using an anhydrous solvent; wet granulating reducing sugar using an anhydrous solvent; and combining the wet granulated 5-amino-2-hydroxybenzoic acid, the wet granulated reducing sugar, and other suitable ingredients to make the pharmaceutical composition. In one embodiment of the method described above, the degradant that does not accumulate above 0.15% (wt/wt) during storage is 5-[2-formyl-5-(hydroxymethyl)-1H-pyrrol-1-yl]-2-hydroxybenzoic acid.

In another embodiment of the method described above, the anhydrous solvent is selected from the group consisting of isopropyl alcohol, acetone, methanol, and ethanol. In another embodiment of the method described above, the anhydrous solvent is isopropyl alcohol.

In another embodiment, the invention provides for methods of making a pharmaceutical composition comprising a safe and effective amount of 5-amino-2-hydroxybenzoic acid and a reducing sugar; that does not accumulate a degradant more than about 0.15% during its about 25°C / 60% relative humidity shelf-life; comprising: dry granulating 5-amino-2-hydroxybenzoic acid and reducing sugar together by a dry granulation process; in which each component is mixed and compressed either by a roller compactor or other heavy-duty compacting equipment, and the resultant compacted material is reduced to the desired particle size; combining granulated ingredients and other suitable ingredients to make the pharmaceutical composition. In one embodiment of the method described above, the degradant that does not accumulate above 0.15% (wt/wt) during storage is 5-[2-Formyl-5-(hydroxymethyl)-1H-pyrrol-1-yl]-2-hydroxybenzoic acid.

In another embodiment, the invention provides for use of a desiccant to prevent formation of 5-[2-Formyl-5-(hydroxymethyl)-1H-pyrrol-1-yl]-2-hydroxybenzoic acid in compositions comprising 5-amino-2-hydroxybenzoic acid, and a reducing sugar. In another embodiment of the use described above, the reducing sugar is lactose.

BRIEF DESCRIPTION OF THE DRAWINGS

Figure 1 shows the effect of relative humidity on generation of degradant 5-[2-Formyl-5-(hydroxymethyl)-1 H-pyrrol-1 -yl]-2-hydroxybenzoic acid.

Figure 2 shows the effect of wet granulation using water or isopropyl alcohol on generation of degradant 5-[2-Formyl-5-(hydroxymethyl)-1H-pyrrol-1-yl]-2-hydroxybenzoic acid.

Figure 3 shows the formation of 5-[2-Formyl-5-(hydroxymethyl)-1H-pyrrol-1-yl]-2-hydroxybenzoic acid during storage at different relative humidities.

Figure 4 shows the formation of 5-[2-Formyl-5-(hydroxymethyl)-1H-pyrrol-1-yl]-2-hydroxybenzoic acid during storage of hermetically sealed packages at various equilibrium humidities.

DETAILED DESCRIPTION OF THE INVENTION

The inventors have now discovered that Asacol™ tablets comprising 5-amino-2-hydroxybenzoic acid and lactose sugar generate degradants. The inventors have discovered that pharmaceutical compositions comprising 5-amino-2-hydroxybenzoic acid and a reducing sugar, e.g., lactose, undergo degradation and produce, in the case of lactose, 5-[2-formyl-5-(hydroxymethyl)-1H-pyrrol-1-yl]-2-hydroxybenzoic acid. The inventors have developed various means to contain and/or reduce the degradation of 5-amino-2-hydroxybenzoic acid.

Stability Testing of New Drug Substances and Products

The International Conference on Harmonization (ICH) of technical requirements for registration of pharmaceuticals for human use has issued guidelines concerning stability testing of new drug substances and products. Regulatory agencies in the United States of America, European Union, and Japan, require that stability testing be carried out on all new drug substances and products and reported according to these guidelines. Relevant portions of the guidelines are reproduced below:

The design of the formal stability studies for the drug product should be based on knowledge of the behavior and properties of the drug substance and from stability studies on the drug substance and on experience gained from clinical formulation studies.

For the long-term studies, the frequency of testing should be sufficient to establish the stability profile of the drug product. For products with a proposed shelf life of at least 12 months, the frequency of testing at the long term storage condition should normally be every 3 months over the first year, every 6 months over the second year, and annually thereafter through the proposed shelf life.

At the accelerated storage condition, a minimum of three time points, including the initial and final time points (e.g., 0, 3, and 6 months), from a 6-month study is recommended. Where an expectation (based on development experience) exists that results from accelerated testing are likely to approach significant change criteria, increased testing should be conducted either by adding samples at the final time point or by including a fourth time point in the study design.

When testing at the intermediate storage condition is called for as a result of significant change at the accelerated storage condition, a minimum of four time points, including the initial and final time points (e.g., 0, 6, 9, 12 months), from a 12-month study is recommended. In general, a drug product should be evaluated under storage conditions (with appropriate tolerances) that test its thermal stability and, if applicable, its sensitivity to moisture or potential for solvent loss. The storage conditions and the lengths of studies chosen should be sufficient to cover storage, shipment, and subsequent use.

Long therm, intermediate, and accelerated storage conditions for drug products are detailed below, where RH means relative humidity.

| Study | Storage condition | Minimum time period covered by data at submission |
|---|---|---|
| Long term | 25°C $\pm$ 2°C/60% RH $\pm$ 5% RH; or 30°C $\pm$ 2°C/65% RH $\pm$ 5% RH | 12 months |
| Intermediate | 30°C $\pm$ 2°C/65% RH $\pm$ 5% RH | 6 months |
| Accelerated | 40°C $\pm$ 2°C/75% RH $\pm$ 5% RH | 6 months |

If long-term studies are conducted at 25°C $\pm$ 2°C/60% RH $\pm$ 5% RH and "significant change" occurs at any time during 6 months' testing at the accelerated storage condition, additional testing at the intermediate storage condition should be conducted and evaluated against significant change criteria. The initial application should include a minimum of 6 months' data from a 12-month study at the intermediate storage condition.

In general, "significant change" for a drug product is defined as:

1. A 5% change in assay from its initial value; or failure to meet the acceptance criteria for potency when using biological or immunological procedures;
2. Any degradation product exceeding its acceptance criterion;
3. Failure to meet the acceptance criteria for appearance, physical attributes, and functionality test (e.g., color, phase separation, re-suspendibility, caking, hardness, dose delivery per actuation); however, some changes in physical attributes (e.g., softening of suppositories, melting of creams) may be expected under accelerated conditions;

and, as appropriate for the dosage form:

4. Failure to meet the acceptance criterion for pH; or
5. Failure to meet the acceptance criteria for dissolution for 12 dosage units.

A systematic approach should be adopted in the presentation and evaluation of the stability information, which should include, as appropriate, results from the physical, chemical, biological, and microbiological tests, including particular attributes of the dosage form (for example, dissolution rate for solid oral dosage forms). One of the major purposes of the stability study is to establish, based on testing a minimum of three batches of the drug product, a shelf life and label storage instructions applicable to all future batches of the drug product manufactured and packaged under similar circumstances. The degree of variability of individual batches affects the confidence that a future production batch will remain within specification throughout its shelf life. Where the data show so little degradation and so little variability that it is apparent from looking at the data that the requested shelf life will be granted, it is normally unnecessary to go through the formal statistical analysis; providing a justification for the omission should be sufficient.

Any evaluation should consider not only the assay but also the degradation products and other appropriate attributes. Where appropriate, attention should be paid to reviewing the adequacy of the mass balance and different stability and degradation performance.

The relevant definitions, provided by the ICH guidelines, are as follows:

Drug substance: The unformulated drug substance that may subsequently be formulated with excipients to produce the dosage form.
Dosage form: A pharmaceutical product type (e.g., tablet, capsule, solution, cream) that contains a drug substance generally, but not necessarily, in association with excipients.
Drug product: The dosage form in the final immediate packaging intended for marketing.

Similarly, the ICH guidelines for the reporting of impurities in new drug products state that it addresses only those impurities in new drug products classified as degradation products of the drug substance or reaction products of the drug substance with an excipient and/or immediate container closure system (collectively referred to as "degradation products" in the guideline). Generally, impurities present in the new drug substance need not be monitored or specified in the new drug product unless they are also degradation products (see ICH Q6A guideline on specifications).
Any degradation product observed in stability studies conducted at the recommended storage condition should be identified when present at a level greater than (>) the identification thresholds described below:

Reporting Thresholds for Degradation Products in New Drug Products

| Maximum Daily Dose | Threshold |
| --- | --- |
| ≤ or equal to 1 g | 0.1% |
| > 1 g | 0.05% |

Degradation product levels may be measured by a variety of techniques, including those that compare an analytical response for a degradation product to that of an appropriate reference standard or to the response of the new drug substance itself. Reference standards used in the analytical procedures for control of degradation products should be evaluated and characterized according to their intended uses. The drug substance may be used to estimate the levels of degradation products. Acceptance criteria and analytical procedures, used to estimate identified or unidentified degradation products, are often based on analytical assumptions (e.g., equivalent detector response).
"Product moisture," as used herein, means the water present within the solid dose form of a composition.
The presence and extent of humidity inside a drug dosage form, or a drug product package, of a composition may be determined in several ways. For packages that contain a desiccant it may be inferred that the package is desiccated if the desiccant appears dry or is an indicating desiccant and has not changed color indicating that it contains excess moisture. Another way is by measuring the products moisture level and comparing it to a calibration curve to determine the equilibrium humidity, which is the amount of "free" or "available" water in a product as opposed to "bound" water. The calibration curve should consist of product moisture level after product has been equilibrated at different humidity levels over a range of interest, (e.g. 10 to 75% RH at 25°C). The product may be considered desiccated if its moisture content corresponds to 30% RH or less at 25°C. Desiccated storage means that the dosage form is exposed to humidity of less than or about 30% relative humidity at 25°C or about 6g of water / kg of air.
Percent water in a composition by weight/weight may be determined by various methods referred to in US Pharmacopoeia (e.g. Sections 921, 731); including, but not limited to, direct titration methods, e.g., Karl Fischer titration or gravimetric methods, e.g., loss on drying (LOD) methods. LOD methods generally, but not exclusively, heat a sample to 105°C, and determine the moisture level by the difference in weight before and after heating.
The quantity of 5-amino-2-hydroxybenzoic acid degradant, 5-[2-Formyl-5-(hydroxymethyl)-1H-pyrrol-1-yl]-2-hydroxybenzoic acid, may be determined by chromatography. A suitable chromatography method is the ion pairing method found in the USP monograph for Modified Release Mesalamine Tablets. The impurities may be detected by the Mesalamine USP method. However, in the chromatogram obtained using USP method, salicylic acid, which is a 5-amino-2-hydroxybenzoic acid impurity and not a degradant, co-elutes with the degradant, 5-[2-Formyl-5-(hydroxymethyl)-1H-pyrrol-1-yl]-2-hydroxybenzoic acid, making quantification more difficult. In a more suitable method, the impurities may be detected by using a Rapid Resolution™ 3.5 μm x 30 mm, C18 cartridge column, and increasing the analyte solution concentration by two-fold.
"Desiccation" is commonly defined as a process of extracting moisture; and "desiccated" is defined as the condition of not comprising or being covered by a liquid (especially water). Desiccation may be achieved in different ways. One way is to reduce the air and / or humidity from the container by physical, chemical, or other means. When handling large quantities of ingredients, it may be suitable to apply vacuum to the container and thereby achieving desiccation. This approach may be unsuitable where repeated openings of container are expected and a vacuum desiccator may not be available every time the container is opened. Another drawback of vacuum desiccation may be that it may not remove all the moisture from the product being desiccated.
Another approach is to use desiccants. A desiccant is a material that will absorb moisture by physical and / or chemical means. Activated desiccants are desiccants that have been treated by heating and ventilating, or by other means, to develop an internal surface on which moisture and certain vapors or gases may collect. Common desiccants include, but are not limited to, silica gel, indicating silica gel, molecular sieves, clay or montmorillonite, activated carbon, and alumina. Indicating silica gel changes color upon absorbing moisture. Many desiccants are commercially available, either in bulk or as packages comprising predetermined amount. Desiccant quantities may be adjusted depending on the need of the application. For example, a bulk product may be stored in a drum with a suitable amount of a desiccant, or it may first be divided into smaller batches and stored appropriately with a smaller quantity of a desiccant. The moisture initially in a package from the drug substance, excipients, and fillers, as well as moisture permeation into the package over its shelf life, will determine the amount of desiccant required in a given package. Fillers include cotton or other fiber, which may be used as a packing material. The quantity of desiccant in an ideal case should be at least sufficient to absorb this moisture and maintain a desiccated environment within the package for the products shelf life. Desiccant Quantity Calculation:

$$\text{Total Moisture to be absorbed by Desiccant (g)} = \text{Product Moisture (g)} + \text{Permeation of moisture over Projected shelf Life in the Package (g)} + \text{Moisture from Package Fillers (g)}.$$

Once the total moisture (g) to be absorbed is calculated, one calculates the desiccant quantity needed as follows:

$$\text{Quantity of Desiccant (g) required} = \frac{\text{Total Moisture to be absorbed by Desiccant (g)}}{\text{Desiccant Capacity (g water / g desiccant)}}.$$

The amount of desiccant required may depend upon the number of unit dosage forms to be packaged per container and the capacity of the desiccant. This calculation governs the predetermined quantity of desiccant to be provided. Normally, the predetermined desiccant quantity is in excess of the desiccant required.

Another approach to achieve reduced water/moisture content in a product is by using solvents other than water in a granulation process. Anhydrous hydrophobic solvents, e.g., isopropyl alcohol, may be used in place of water in a wet granulation. The drug substance or reducing sugar should not be soluble in the solvent and the solvent should have a sufficiently low boiling point to allow for efficient drying from the product. Examples of suitable solvents include, but are not limited to, isopropyl alcohol, acetone, methanol, and ethanol.

The pharmaceutical compositions of the invention comprise a safe and effective amount of 5-amino-2-hydroxybenzoic acid, a reducing sugar; and pharmaceutically acceptable carriers or excipients.

A "safe and effective amount" of a compound is an amount that is effective, to treat a disorder, without undue adverse side effects (such as toxicity, irritation, or allergic response), commensurate with a reasonable benefit/risk ratio when used in the manner of this invention. The specific "safe and effective amount" will vary with such factors as the particular condition being treated, the physical condition of the patient, the duration of treatment, the nature of concurrent therapy (if any), the specific dosage form to be used, the excipients employed, the solubility of the subject compound therein, and the dosage regimen desired for the composition.

The term "pharmaceutically acceptable carrier", as used herein, means one or more compatible diluents or encapsulating substances, which are suitable for administration to an animal, preferably a mammal, more preferably a human.

The term "compatible", as used herein, means that the components of the composition are capable of being commingled with the subject compound, and with each other, in a manner such that there is minimal interaction that would substantially reduce the pharmaceutical efficacy of the composition under ordinary use situations. Pharmaceutically acceptable carriers should be of sufficiently high purity and sufficiently low toxicity to render them suitable for administration to the subject, preferably a mammal, more preferably a human being treated.

Some examples of substances which may serve as pharmaceutically acceptable carriers or components thereof are: reducing and non-reducing sugars; starches; cellulose, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as TWEEN™, wetting agents, such sodium lauryl sulfate; coloring agents; flavoring agents; tableting agents, stabilizers; antioxidants; preservatives.

The choice of a pharmaceutically acceptable carrier to be used in conjunction with the subject compound is determined with consideration for the compound to be administered, and is within the ambit of the skilled person.

The compositions of this invention may be provided in unit dosage form. As used herein, a "unit dosage form" is a composition of this invention comprising an amount of a subject compound that is suitable for administration to a subject according to good medical practice. These compositions preferably contain from about 5 mg (milligrams) to about 1000 mg of a composition of the invention.

Various oral dosage forms may be used, including such solid forms as tablets, capsules, granules, and bulk powders. Tablets may be compressed, tablet triturates, enteric-coated, sugarcoated, film-coated, or multiple-compressed, comprising suitable binders, lubricants, diluents, disintegrating agents, coloring agents, flavoring agents, flow-inducing agents, and melting agents.

Such compositions may also be coated by conventional methods, suitably with pH or time-dependent coatings, such that the subject compound is released in the gastrointestinal tract in the vicinity of the desired topical application, or at various times to extend the desired action. Such dosage forms suitably include, but are not limited to, one or more of cellulose acetate phthalate, polyvinylacetate phthalate, hydroxypropyl methylcellulose phthalate, ethyl cellulose, Eudragit™ coatings, waxes, and shellac.

Also disclosed are kits comprising at least one unit dosage form comprising a safe and effective amount of 5-amino-2-hydroxybenzoic acid and a reducing sugar; wherein the concentration of a degradant that may accumulate during storage in the unit dosage form is no more than about 0.15%, when measured according to a stability testing method recognized by International Conference on Harmonization of technical requirements for registration of pharmaceuticals; further comprising predetermined amount of a desiccant. In order to control degradation, the desiccant and the unit dosage form may need to share the same atmosphere. This may be achieved using various packaging combinations. In one embodiment, a predetermined amount of a desiccant is placed inside of a bottle along with a predetermined quantity of unit dosage form. The bottle may contain a foil seal to limit the moisture permeation over the drug product shelf life or until the bottle is opened.

In another embodiment, a blister card may be formed with multiple cavities wherein one or more of the cavities may comprise a desiccant. Small channels may be formed between the cavity comprising desiccant, and other cavities comprising a unit dosage form, thereby allowing the desiccant to absorb moisture from each of the cavities on the blister card. The blister card may be made from aluminum foil or other suitable barrier materials.

In one embodiment, a blister card could be formed with each cavity for a unit dosage form having an adjoining cavity that would contain desiccant. The blister card would ideally be made from aluminum foil but could be made from other barrier materials as well.

In another embodiment, a pouch may be manufactured comprising one or more unit dosage form and desiccant. The pouch may be made from aluminum foil or other suitable barrier materials.

In one embodiment, a bottle may be used to contain at least one unit dosage form and a desiccant. Desiccant may be provided in form of a sachet, a lining or other suitable means. The bottle may have a metal or other barrier material, closure and may contain a seal. The bottle may be made of glass, plastic, metal, e.g., aluminum, or other suitable material.

EXAMPLES

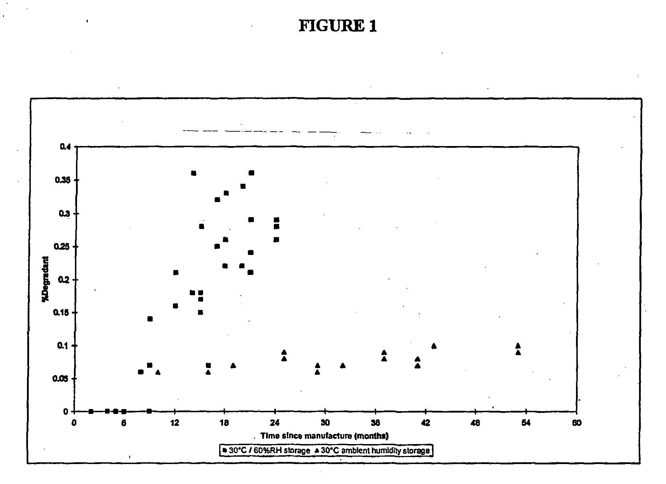

Example 1. Effect of Humidity on Generation of 5-[2-Formyl-5-(hydroxymethyl)-1H-pyrrol-1-yl]-2-hydroxybenzoic acid. Several batches of 5-amino-2-hydroxybenzoic acid and lactose are monitored at conditions of 30°C and ambient humidity (varies with the geographical location of the experiment but usually within 10-35% RH at 30°C for a laboratory setting) and 30°C and 60% RH for up to 54 months. Concentration of the degradant 5-[2-Formyl-5-(hydroxymethyl)-1H-pyrrol-1-yl]-2-hydroxybenzoic acid in samples is measured at various time points using HPLC. It may be observed in Figure 1 that the higher relative humidity of 60% leads to accelerated generation of the degradant 5-[2-Formyl-5-(hydroxymethyl)-1H-pyrrol-1-yl]-2-hydroxybenzoic acid than at ambient humidity condition.

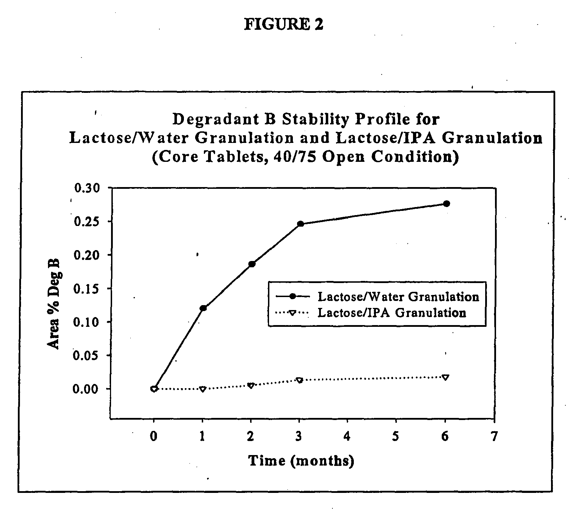

Example 2. Effect of Anhydrous Solvent Wet Granulation versus Aqueous Wet Granulation on Generation of 5-[2-Formyl-5-(hydroxymethyl)-1H-pyrrol-1-yl]-2-hydroxybenzoic acid. A solvent, such as isopropyl alcohol, may be used in place of water in a wet granulation. Two identical formulations comprising 5-amino-2-hydroxybenzoic acid and lactose are wet granulated using either water or isopropyl alcohol (IPA). Both granulations are then oven dried to <1% product moisture as measured by LOD and are stored at 40°C constant temperature and 75% RH humidity chamber for 6 months. Figure 2 shows that generation of the degradant is accelerated when the product is synthesized using aqueous wet granulation. On the other hand, granulation produced with IPA shows significantly less degradation.

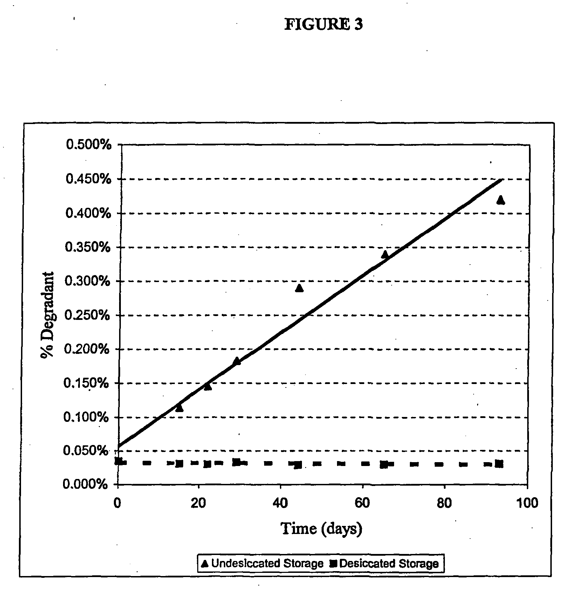

Example 3. Tablet Degradation during Storage at Different Relative Humidity. Identical tablets comprising 5-amino-2-hydroxybenzoic acid and lactose are placed in a 40°C constant temperature chamber desiccated, or under 75% relative humidity. The tablets stored at 75% RH (humidified) show significant degradation after 90 days of storage, while the tablets stored in a desiccated container show no degradation as shown in Figure 3. The desiccated container maintains product moisture levels of about 1.1% or less throughout the duration of the experiment. The undesiccated container absorbs additional moisture from the environment and ranges from 1.1% to 1.8% product moisture throughout the study.

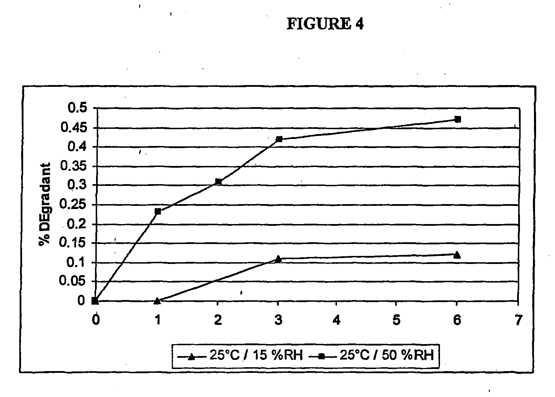

Example 4. Tablet Degradation in Hermetically-Sealed Packages at Various Equilibrium Humidities. Identical dosage forms are sealed in hermetic (moisture impervious) foil (foil blister packages), after equilibrating them for product moisture content at 15 and 50 %RH prior to being packaged The product equilibrated at the lower humidity demonstrates a reduced degradation rate compared to the product that is equilibrated at higher humidity (Figure 4).

Example 5. A kit comprising 5-amino-2-hydroxybenzoic acid (Asacol™) Each unit dosage form contains:

| | |
|---|---|
| 5-amino-2-hydroxybenzoic acid | 400 mg |
| Lactose granules | 76 mg |

and other suitable excipients.

The unit dosage forms of the compositions are packaged in a suitable container comprising the following amounts of desiccant:

| | |
|---|---|
| 180 unit doses (Asacol™) | with about 6 g desiccant silica gel; |
| 60 unit doses (Asacol™) | with about 4 g desiccant silica gel; and |
| 12 unit doses (Asacol™) | with about 1.5 g desiccant silica gel. |

Example 6. A kit comprising 5-amino-2-hydroxybenzoic acid (Asacol™) Each unit dosage form contains:

| | |
|---|---|
| 5-amino-2-hydroxybenzoic acid | 800 mg |
| Lactose granules | 76.4 mg |

and other suitable excipients.

The unit dosage forms of the compositions are packaged in a suitable container comprising the following amounts of desiccant:

| | |
|---|---|
| 12 unit doses (Asacol™) | with about 4 g desiccant silica gel; |
| 180 unit doses (Asacol™) | with about 10 g desiccant silica gel; and |
| 36 unit doses (Asacol™) | with about 4 g desiccant silica gel. |

Example 7. A suitable bottle or a container for packaging a preferred number of unit dosage forms may exhibit the following properties:

Natural high-density polyethylene (HDPE) bottle;

Closure: white, child-resistant, HDPE outer shell with polypropylene inner shell and foil induction seal. It may optionally contain cotton.

Except as, otherwise noted, all amounts including quantities, percentages, portions, and proportions, are understood to be modified by the word "about," and amounts are not intended to indicate significant digits.

Except as otherwise noted, the articles "a", "an", and "the" mean "one or more".

## Claims

1. A kit **characterized in that** it comprises:

   (a) at least one unit dosage form comprising:

      (i) a safe and effective amount of 5-amino=2-hydroxybenzoic acid;
      (ii) a reducing sugar; and

(b) a predetermined amount of a desiccant.

2.  The kit of claim 1, wherein the concentration of a degradant that may accumulate during storage in the unit dosage form is no more than 0.15%.

3.  The kit of claim 1 or 2, wherein the unit dosage form is made via wet-granulation using water.

4.  The kit of claim 2, wherein the degradant is 5-[2-Formyl-5-(hydroxymethyl)-1H-pyrrol-1-yl]-2-hydroxybenzoic acid.

5.  The kit of any one of the preceding claims, wherein at least one unit dosage form and the desiccant are placed in a re-sealable or re-closable container.

6.  The kit of any one of the preceding claims, wherein at least one unit dosage form is placed in one or more cavities while the desiccant is placed in separate but connected cavities sharing the same environment with the unit dosage form.

7.  The kit of any one of the preceding claims, wherein the reducing sugar is selected from the group consisting of lactose, glucose, galactose, and sucrose.

8.  The kit of any one of the preceding claims, wherein the desiccant is an activated desiccant.

9.  The kit of claim 8, wherein the activated desiccant is selected from the group consisting of silica gel, indicating silica gel, molecular sieve, clay, montmorillonite, carbon, alumina, and mixtures thereof.

10. A method of making a composition comprising 5-amino-2-hydroxybenzoic acid and a reducing sugar, that does not accumulate more than 0.15% of the degradant 5-[2-Formyl-5-(hydroxymethyl)-1H-pyrrol-1-yl]-2-hydroxybenzoic acid during its proposed shelf life; **characterized in that** it comprises the steps of:

    (a) wet granulating 5-amino-2-hydroxybenzoic acid and a reducing sugar by a process using an anhydrous solvent;
    (b) drying the wet granulated mixture;
    (c) combining the dried granulation mixture (b) and other excipients to make the composition.

11. The method of claim 10, wherein the anhydrous solvent is selected from the group consisting of isopropyl alcohol, acetone, methanol, and ethanol.

12. A method of making a composition comprising 5-amino-2-hydroxybenzoic acid and a reducing sugar, that does not accumulate more than about 0.15% of the degradant 5-[2-Formyl-5-(hydroxymethyl)-1H-pyrrol-1-yl]-2-hydroxybenzoic acid during its about 25°C / 60% relative humidity shelf life;
    **characterized in that** it comprises:

    (a) combining 5-amino-2-hydroxybenzoic acid, and a reducing sugar together and granulating them by a dry granulation process; and
    (b) combining granulated ingredients and other suitable excipients to make the composition.

13. Use of a desiccant to prevent formation of 5-[2-Formyl-5-(hydroxymethyl)-1H-pyrrol-1-yl]-2-hydroxybenzoic acid in compositions comprising 5-amino-2-hydroxybenzoic acid, and a reducing sugar.

**Patentansprüche**

1.  Set, das **dadurch gekennzeichnet ist, dass** es Folgendes umfasst:

    (a) mindestens eine Einheitsdosierungsform, die Folgendes umfasst:

        (i) eine sichere und wirksame Menge an 5-Amino-2-hydroxybenzoesäure;
        (ii) einen reduzierenden Zucker; und

(b) eine vorgegebene Menge eines Trocknungsmittels.

2. Set nach Anspruch 1, wobei die Konzentration eines Degradanten, der sich während der Lagerzeit in der Einheitsdosierungsform ansammeln kann, höchstens 0,15 % beträgt.

3. Set nach Anspruch 1 oder 2, wobei die Einheitsdosierungsform über Nassgranulation unter Verwendung von Wasser hergestellt wird.

4. Set nach Anspruch 2, wobei der Degradant 5-[2-Formyl-5-(hydroxymethyl)-1H-pyrrol-1-yl]-2-hydroxybenzoesäure ist.

5. Set nach einem der vorstehenden Ansprüche, wobei mindestens eine Einheitsdosierungsform und das Trocknungsmittel in einen wiederversiegelbaren oder wiederverschließbaren Behälter gegeben werden.

6. Set nach einem der vorstehenden Ansprüche, wobei mindestens eine Einheitsdosierungsform in eine oder mehrere Vertiefungen gegeben wird, während das Trocknungsmittel in separate, jedoch verbundene Vertiefungen gegeben wird, die sich in der gleichen Umgebung wie die Einheitsdosierungsform befinden.

7. Set nach einem der vorstehenden Ansprüche, wobei der reduzierende Zucker ausgewählt ist aus der Gruppe, bestehend aus Lactose, Glucose, Galactose und Saccharose.

8. Set nach einem der vorstehenden Ansprüche, wobei das Trocknungsmittel ein aktiviertes Trocknungsmittel ist.

9. Set nach Anspruch 8, wobei das aktivierte Trocknungsmittel ausgewählt ist aus der Gruppe, bestehend aus Kieselgel, Kieselgel mit Feuchtigkeitsindikator, Molekularsieb, Ton, Montmorillonit, Kohlenstoff, Aluminiumoxid und Mischungen davon.

10. Verfahren zum Herstellen einer Zusammensetzung, die 5-Amino-2-hydroxybenzoesäure und einen reduzierenden Zucker umfasst und in der sich höchstens 0,15 % des Degradanten 5-[2-Formyl-5-(hydroxymethyl)-1H-pyrrol-1-yl]-2-hydroxybenzoesäure während ihrer empfohlenen Haltbarkeitsdauer ansammeln; **dadurch gekennzeichnet, dass** es folgende Schritte umfasst:

   (a) Nassgranulation von 5-Amino-2-hydroxybenzoesäure und einem reduzierenden Zucker durch ein Verfahren, das ein wasserfreies Lösungsmittel verwendet;
   (b) Trocknen der nassgranulierten Mischung;
   (c) Vermischen der getrockneten Granulatmischung (b) und anderer Arzneimittelträger, um die Zusammensetzung herzustellen.

11. Verfahren nach Anspruch 10, wobei das wasserfreie Lösungsmittel ausgewählt ist aus der Gruppe, bestehend aus Isopropylalkohol, Aceton, Methanol und Ethanol.

12. Verfahren zum Herstellen einer Zusammensetzung, die 5-Amino-2-hydroxybenzoesäure und einen reduzierenden Zucker umfasst und in der sich höchstens etwa 0,15 % des Degradanten 5-[2-Formyl-5-(hydroxymethyl)-1H-pyrrol-1-yl]-2-hydroxybenzoesäure während ihrer Haltbarkeitsdauer bei ungefähr 25 °C / 60 % relativer Luftfeuchte ansammeln; **dadurch gekennzeichnet, dass** es folgende Schritte umfasst:

   (a) Vermischen von 5-Amino-2-hydroxybenzoesäure und einem reduzierenden Zucker und Granulieren beider durch ein Trockengranulationsverfahren; und
   (b) Vermischen der granulierten Bestandteile und anderer geeigneter Arzneimittelträger, um die Zusammensetzung herzustellen.

13. Verwendung eines Trocknungsmittels zum Verhindern der Bildung von 5-[2-Formyl-5-(hydroxymethyl)-1H-pyrrol-1-yl]-2-hydroxybenzoesäure in Zusammensetzungen, die 5-Amino-2-hydroxybenzoesäure umfassen, und eines reduzierenden Zuckers.

**Revendications**

1. Trousse **caractérisée en ce qu'**elle comprend :

    (a) au moins une forme pharmaceutique unitaire comprenant :

        (i) une quantité sûre et efficace d'acide 5-amino-2-hydroxybenzoïque ;
        (ii) un sucre réducteur ; et

    (b) une quantité prédéterminée d'un déshydratant.

2. Trousse selon la revendication 1, dans laquelle la concentration d'un dégradant qui peut s'accumuler durant le stockage dans la forme pharmaceutique unitaire ne représente pas plus de 0,15 %.

3. Trousse selon la revendication 1 ou 2, dans laquelle la forme pharmaceutique unitaire est fabriquée via une granulation humide en utilisant de l'eau.

4. Trousse selon la revendication 2, dans laquelle le dégradant est de l'acide 5-[2-formyl-5-(hydroxyméthyl)-1H-pyrrol-1-yl]-2-hydroxybenzoïque.

5. Trousse selon l'une quelconque des revendications précédentes, dans laquelle au moins une forme pharmaceutique unitaire et le déshydratant sont placés dans un conteneur re-scellable ou re-fermable.

6. Trousse selon l'une quelconque des revendications précédentes, dans laquelle au moins une forme pharmaceutique unitaire est placée dans une ou plusieurs cavités alors que le déshydratant est placé dans des cavités indépendantes mais reliées partageant le même environnement avec la forme pharmaceutique unitaire.

7. Trousse selon l'une quelconque des revendications précédentes, dans laquelle le sucre réducteur est choisi dans le groupe constitué de lactose, glucose, galactose et saccharose.

8. Trousse selon l'une quelconque des revendications précédentes, dans laquelle le déshydratant est un déshydratant activé.

9. Trousse selon la revendication 8, dans laquelle le déshydratant activé est choisi dans le groupe constitué de gel de silice, gel de silice indicateur, tamis moléculaire, argile, montmorillonite, carbone, alumine et leurs mélanges.

10. Procédé de fabrication d'une composition comprenant de l'acide 5-amino-2-hydroxybenzoïque et un sucre réducteur, qui n'accumule pas plus de 0,15 % de l'acide 5-[2-formyl-5-(hydroxyméthyl)-1H-pyrrol-1-yl]-2-hydroxybenzoïque dégradant durant sa durée de conservation proposée ; **caractérisé en ce qu'**il comprend les étapes consistant à :

    (a) granuler en voie humide de l'acide 5-amino-2-hydroxybenzoïque et un sucre réducteur par un procédé utilisant un solvant anhydre ;
    (b) sécher le mélange granulé humide ;
    (c) combiner le mélange de granulation séché (b) et d'autres excipients pour fabriquer la composition.

11. Procédé selon la revendication 10, dans lequel le solvant anhydre est choisi dans le groupe constitué d'alcool isopropylique, acétone, méthanol et éthanol.

12. Procédé de fabrication d'une composition comprenant de l'acide 5-amino-2-hydroxybenzoïque et un sucre réducteur, qui n'accumule pas plus d'environ 0,15 % de l'acide 5-[2-formyl-5-(hydroxyméthyl)-1H-pyrrol-1-yl]-2-hydroxybenzoïque dégradant durant sa durée de conservation à environ 25 °C / 60 % d'humidité relative ; **caractérisé en ce qu'**il comprend :

    (a) la combinaison d'acide 5-amino-2-hydroxybenzoïque, et d'un sucre réducteur conjointement et leur granulation par un procédé de granulation à sec ; et
    (b) la combinaison des ingrédients granulés et d'autres excipients appropriés pour fabriquer la composition.

13. Utilisation d'un déshydratant pour empêcher la formation d'acide 5-[2-formol-5-(hydroxyméthyl)-1H-pyrrol-1-yl]-2-hydroxybenzoïque dans des compositions comprenant de l'acide 5-amino-2-hydroxybenzoïque, et un sucre réducteur.

FIGURE 1

FIGURE 2

**Degradant B Stability Profile for
Lactose/Water Granulation and Lactose/IPA Granulation
(Core Tablets, 40/75 Open Condition)**

FIGURE 3

**FIGURE 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI8199815032 B


**Non-patent literature cited in the description**

- **SMITH et al.** *Proc. Natl. Acad. Sci. USA,* 1993, vol. 91, 5710-5714
- **NELSON ; LABUZA.** *J. Food Eng.,* 1994, vol. 22, 271-289
- **JENSEN et al.** *Int. J. Pharmaceutics,* 1992, vol. 88, 177-187